# EUROPEAN PATENT SPECIFICATION

(11) **EP 1 951 364 B1**
(45) Date of publication and mention of the grant of the patent: **02.01.2013**
(21) Application number: 06838155.7
(22) Date of filing: 20.11.2006
(51) Int. Cl.: A61N 1/372

(54) **METHOD AND APPARATUS FOR ANALYTE DATA TELEMETRY**
VERFAHREN UND VORRICHTUNG ZUR ANALYTDATEN-TELEMETRIE
PROCEDE ET DISPOSITIF PERMETTANT DE MESURER A DISTANCE DES DONNEES RELATIVES A DES SUBSTANCES A ANALYSER

(30) Priority: 22.11.2005 US 739148 P; 24.10.2006 US 552222
(43) Date of publication of application: 06.08.2008
(73) Proprietor: Isense Corporation, Wilsonville, OR 97070 (US)
(72) Inventor: BRUCE, Robert, Beaverton, OR 97007 (US); NEINAST, Mark, Lake Oswego, OR 97034 (US); WARD, W., Kenneth, Portland, OR 97231 (US); HOUSE, Jody, Hillsboro, OR 97123 (US)
(74) Representative: Moore, Barry
(86) International application number: PCT/US2006/045016
(87) International publication number: WO 2007/062013

(56) References cited:
- EP-A- 1 151 759
- WO-A-2004/061420
- US-A- 5 507 288
- US-A- 6 067 474
- US-A1- 2001 051 766
- US-A1- 2001 051 766
- US-A1- 2002 002 326
- US-A1- 2002 087 203
- US-A1- 2003 161 411
- US-A1- 2004 059 391
- US-A1- 2004 171 921
- US-A1- 2004 171 921
- US-A1- 2005 075 696
- US-A1- 2005 075 696
- US-A1- 2005 245 795
- US-A1- 2005 245 795
- US-B1- 6 450 172
- US-B1- 6 809 653

## Description

### Technical Field

Embodiments of the present invention relate to the field of telemetry, more specifically, to methods and apparatuses for providing telemetry in association with medical devices.

### Background

Medical telemetry systems perform measurements of one or more patient parameters of medical interest. The measurement data may be transmitted to a remote location where it may be monitored and recorded. Generally, the data transmission is accomplished without the use of wires between the measurement location and the monitoring/recording location.

In many cases the measurement and data transmission portion of a telemetry system may be attached to a patient and operate while the patient is ambulatory. Therefore, there is value in reducing the size and weight of the telemetry measurement and transmission system. Also, in many cases, measurements may be made over a period of time. This feature suggests a need for low power consumption in the data measurement and transmission device so that it may be operated on batteries, for example, without compromising the size and weight constraints. US 2001 0051766 relates to an endoscopic device which is introduced into the intestinal tract of a living organism and which operates to obtain, store or transmit one or more types of data as visual data.

### Brief Description of the Drawings

Embodiments of the present invention will be readily understood by the following detailed description in conjunction with the accompanying drawings. Embodiments of the invention are illustrated by way of example and not by way of limitation in the figures of the accompanying drawings.
Figure 1 illustrates a low-power SAW-stabilized RF transmitter in accordance with an embodiment of the present invention;
Figure 2 illustrates an RF transmitter using Direct-Sequence Spread-Spectrum on an audio sub-carrier in accordance with an embodiment of the present invention;
Figure 3 illustrates an RF receiver using Direct-Sequence Spread-Spectrum on an audio sub-carrier in accordance with an embodiment of the present invention; and
Figure 4 illustrates an exemplary telemetry arrangement for use with a biosensor in accordance with an embodiment of the present invention.

### Detailed Description of Embodiments of the Invention

In the following detailed description, reference is made to the accompanying drawings which form a part hereof, and in which is shown by way of illustration embodiments in which the invention may be practiced. It is to be understood that other embodiments may be utilized and structural or logical changes may be made without departing from the scope of the present invention. Therefore, the following detailed description is not to be taken in a limiting sense, and the scope of embodiments in accordance with the present invention is defined by the appended claims and their equivalents.

Various operations may be described as multiple discrete operations in turn, in a manner that may be helpful in understanding embodiments of the present invention; however, the order of description should not be construed to imply that these operations are order dependent.

The description may use perspective-based descriptions such as up/down, back/front, and top/bottom. Such descriptions are merely used to facilitate the discussion and are not intended to restrict the application of embodiments of the present invention.

For the purposes of the present invention, a phrase in the form "A/B" means A or B. For the purposes of the present invention, a phrase in the form "A and/or B" means "(A), (B), or (A and B)". For the purposes of the present invention, a phrase in the form "at least one of A, B, and C" means "(A), (B), (C), (A and B), (A and C), (B and C), or (A, B and C)". For the purposes of the present invention, a phrase in the form "(A)B" means "(B) or (AB)" that is, A is an optional element.

The description may use the phrases "in an embodiment," or "in embodiments," which may each refer to one or more of the same or different embodiments. Furthermore, the terms "comprising," "including," "having," and the like, as used with respect to embodiments of the present invention, are synonymous.

Embodiments of the present invention provide methods and apparatuses for telemetry, more specifically, to methods and apparatuses for providing telemetry in association with medical devices. Embodiments of the present invention provide continuous telemetry between a transmitter and a monitoring unit, for example in a medical monitoring environment.

In an embodiment of the present invention, a transcutaneous biosensor may be used to measure blood glucose levels in a patient for the management of diabetes. In an embodiment, a sensor control unit may be attached to a body and connected to a partially or fully implanted sensor. In an embodiment, a sensor control unit may remain in continuous operation for a period of days, a week, or more. Because a patient may wear the sensor control unit during normal daily activities, small size and freedom from maintenance activities, such as changing batteries, may be valued features.

In an embodiment of the present invention, a sensor control unit digitizes data from a sensor and transmits the data using telemetry to a separate monitoring unit. The monitoring unit may display glucose values, record and store historical glucose data, and/or provide an alarm or other indication of one or more conditions of medical significance.

In an embodiment of the present invention, telemetry may be provided via inductive coupling of two or more magnetic field producing bodies. In an embodiment of the present invention, telemetry may be provided via infra-red communication between two or more bodies.

In an embodiment, telemetry may be, for example, radio-frequency (RF), and may be transmitted, for example, in the 915 MHz ISM (Industrial, Scientific, Medical) band, in the Ultra-High-Frequency (UHF) region of the RF spectrum. RF transmission may be provided in any other suitable frequency, such as 868MHz or 2.4GHz, etc.

In an embodiment of the present invention, when data is transmitted to a remote location by RF signaling, the RF transmitter may provide adequate RF transmission for reliable reception across the distance between the transmitter and the receiving (monitoring/recording) device. In some embodiments, the amount of power consumed by the RF transmitter may be a major portion of total power consumption in the data transmitting device and thus may impose limitations on battery life and/or size and weight.

Embodiments of the present invention address the problem of low battery life by operating an RF transmitter intermittently for short periods of time, so that the average power consumed by the transmitter may be kept low. Nevertheless, intermittent medical data transmission is not the only solution, and may not be best in some situations. When data is stored or buffered in a measurement device, a variable interval of time elapses between when a given measurement is made at the patient and the time when this measurement data is available at the remote location for monitoring and recording. In an alarm condition, for example, data may need to be transmitted without delay and continuous data may need to be transmitted to adequately monitor the patient condition.

Thus, an embodiment of the present invention provides for continuous data transmission.

A type of continuous transmission was described in Shichiri et al., Telemetry Glucose Monitoring Device with Needle-type Glucose Sensor, Diabetes Care Vol. 9, No. 3, May-June 1986, which discloses a wireless glucose monitoring system using continuous RF telemetry. Shichiri converts a variable analog current from a glucose sensor to a corresponding variable high-frequency audio signal. This is accomplished by applying the variable current to a current-to-voltage converting amplifier. The resulting variable voltage is applied as input to a voltage-frequency converter. The resulting high-frequency audio signal is applied as modulation to a VHF frequency-modulated RF transmitter which operates continuously. A suitable VHF FM receiver demodulates the signal yielding a replica of the variable high-frequency audio signal. The replica variable high-frequency audio signal is applied to a frequency-to-voltage converter to yield a variable voltage corresponding to the current at the glucose sensor. At the receiver, the variable analog voltage can be recorded, displayed and/or converted to digital format for further processing and storage.

An embodiment of the present invention also uses an audio signal to modulate a RF transmitter to convey measurement data from a glucose (or other analyte) sensor telemetrically. However, such an embodiment of the present invention differs from the approach disclosed by Shichiri in that a variable voltage, corresponding to a glucose level measured by a sensor, is quantized and converted to a binary digital representation of the variable voltage before being applied as modulation to a FM RF transmitter. Since data is transmitted digitally, an embodiment of the present invention may transmit, in addition to the digital representation of a glucose level measured by the sensor, other digital information such as data identifying the specific RF transmitter, error detection and/or error correction data. An embodiment of the present invention may also allow processing of the digitally transmitted data to provide improvement in the precision and accuracy of transmitted glucose sensor data and improvements in the ability of the receiver to correctly receive the data in the presence of noise or interfering RF signals.

An embodiment of the present invention thus provides a scheme that allows medical telemetry data to be transmitted continuously, for example using RF transmission. An embodiment of the present invention provides continuous transmission without substantially increasing average power consumption of an RF transmitter as compared to an intermittent transmission approach. An embodiment of the present invention allows multiple RF transmitters to transmit data continuously, in close proximity to each other, and without causing interference between or among the multiple data transmissions.

In an embodiment of the present invention, the amount of power consumed by a continuous telemetry transmitter may be reduced significantly so that the transmitter consumes an amount of power comparable to an intermittently-operating transmitter, while providing the benefits of continuously transmitting data. In an embodiment of the present invention, low power consumption allows for continuous telemetry transmission with an ambulatory patient.

In an embodiment of the present invention, a data transmission scheme may provide reliable data transfer under a range of normal operating conditions, including situations in which multiple transmitters are operating on the same RF frequency and/or within range of the receiver.

A multiple transmitter environment may arise with separate transmission systems, or in a designed system utilizing multiple transmitters. In an embodiment of the present invention, multiple transmitters may be used in a single unit, or in a system having multiple electrically or telemetrically connected units. For example, a first transmitter may be used for transmitting identification data while a second transmitter may be used for transmitting analyte-dependent data.

In an embodiment, an RF transmitter may be provided that consumes very low power while outputting a continuous, low power signal, for example 50 to 100 microwatts. In an embodiment, a UHF RF transmitter may include a frequency synthesizer or Phase-Locked-Loop (PLL) frequency multiplier to allow a high-frequency RF carrier to be generated using a low-cost low-frequency quartz crystal as the frequency reference. In an embodiment of the present invention, the synthesizer stage may be followed by an RF power amplifier.

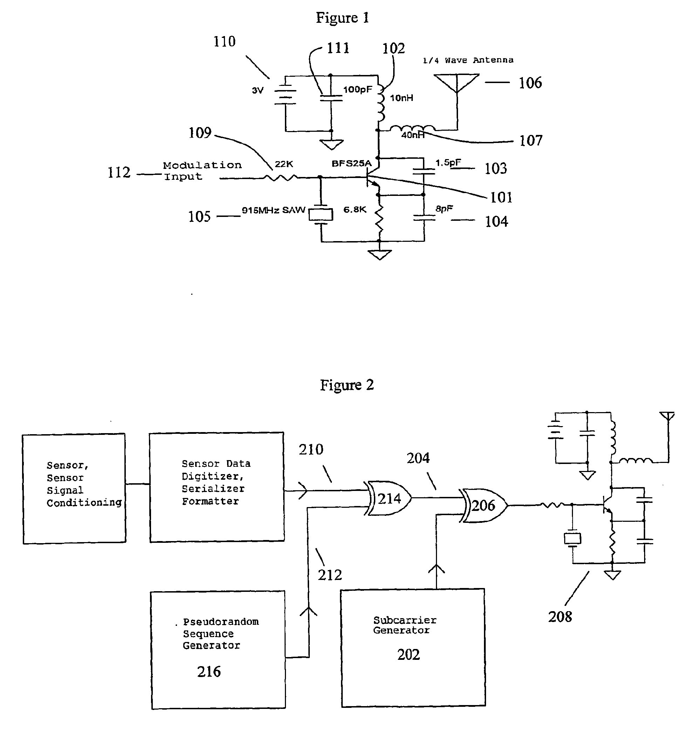

In an embodiment of the present invention, a SAW (surface acoustic wave) resonator may be configured with a transistor oscillator to generate the UHF RF carrier frequency directly without the usual frequency synthesis stage. The transmitted RF signal may be obtained directly from the transistor oscillator without an additional RF power amplifier (Figure 1).

Figure 1 illustrates a low-power SAW stabilized RF transmitter in accordance with an embodiment of the present invention. As shown in Figure 1, the transmitter comprises a Colpitts oscillator, using transistor 101 to provide gain to sustain oscillations. Inductor 102 and capacitors 103 and 104 form a resonant circuit at the desired transmission frequency. Capacitor 111 is an RF bypass capacitor, providing an RF path to ground for the battery-end of inductor 102, thus completing the resonant circuit. The circuit formed by 102, 103, and 104 also provides a phase shift between the signal which appears at the collector of transistor 101 and the emitter of transistor 101. This phase shift and the amplification provided by transistor 101 provides positive feedback and gain to sustain oscillation at the resonant frequency of the circuit formed by 102, 103, and 104, provided there is a low impedance path to ground from the base of transistor 101. SAW resonator 105 provides the required low impedance path to ground at its resonant frequency. SAW resonator 105 provides its low impedance path at a well-defined and stable frequency, thus assuring that oscillations occur at a desired frequency and with high stability to variations in temperature and power supply voltage. In an embodiment, a portion of the oscillator output is coupled to an antenna 106 through inductor 107, which limits the amount of RF energy that is coupled away from the oscillator by the antenna, assuring reliable operation. Transistor 101 is biased to provide the required gain and RF power output by resistors 108 and 109. Resistor 109 provides base current to transistor 101 when a positive voltage is applied at modulation input 112. Increasing voltage at this point increases base current and thus transistor collector current and RF output, allowing the strength of the RF signal to be varied for modulation. Resistor 108, the emitter resistor, stabilizes transistor collector-to-emitter current. Emitter resistor 108 also sets the maximum power level for the oscillator. A lower resistance value allows a higher RF power level from the transmitter, along with higher power consumption from battery 110. A higher resistance value at resistor 108 reduces transmitter RF power level and also reduces transmitter power consumption from battery 110 for longer battery life. In an embodiment, with the selected value for resistor 108, the transmitter will operate consuming less than 0.25 milliamps from the battery 110.

The output of a low power RF transmitter may be modulated in accordance with embodiments of the present invention in several ways so that data may be transmitted. First, some degree of frequency modulation may be possible by electronically switching an additional loading capacitor across a SAW resonator. For example, see US Patent No. 5,793,261, the entire disclosure of which is hereby incorporated by reference, for details regarding a suitable method to perform frequency modulation with a SAW resonator for use in various embodiments of the present invention. Second, the amplitude of the transmitter output may be modulated by adjusting the base bias to the transistor oscillator. Binary on-off modulation may be obtained or the amplitude may be varied in a continuous way by varying the base bias voltage.

If a transmitter is to be continuously operated for extended periods of time, in an embodiment, one or more rechargeable or disposable batteries may be provided. In an embodiment, a battery may be recharged with power provided by inductive coupling to allow the battery to be recharged while the unit is in operation and attached to the patient, without electrical connections from the battery to the charging power source. In an embodiment of the present invention, a battery may be recharged by coupling the battery with a separate charging unit or docking station. In a system in accordance with an embodiment of the present invention, a plurality of batteries may be provided of which at least one of the plurality of batteries may be disposable and at least one of the plurality of batteries may be rechargeable.

An embodiment of the present invention provides a method for transmitting data in a way that a properly configured receiver may receive signals without interference from an undesired transmitter when one or more transmitters are operating on the same frequency and/or within receiving range. Embodiments of the present invention may also provide additional security for a patient in discouraging the detection or recovery of personal medical data by unauthorized parties, as compared to conventional approaches.

In an embodiment, data from different transmitters may be transmitted at different power levels or signal amplitudes to differentiate one transmitter from the next. For example, a first transmitter may transmit a series of data points at nA (nanoamps), pA (picoamps), fA (femtoamps), nA, pA, fA levels. A second transmitter may, for example, transmit a series of data points at nA, nA, pA, nA, nA, pA levels. The pattern thus may be used to identify the particular transmitter based on the uniqueness or differentiation of the pattern. Thus, for purposes of the present invention, the term "transmission pattern" refers to the regular order and signal strength/amplitude of transmitted data other than continuously at the same signal strength/amplitude.

In an embodiment, data may be transmitted using Spread Spectrum techniques applied to an audio sub-carrier, which in turn may be used to amplitude modulate the RF transmitter. This embodiment allows recovery of data from one or more transmitters operating on the same frequency within range of a receiver. This embodiment is also suited to application in a low-power telemetry transmission system. Such an embodiment of the present invention may be implemented with very low-power circuitry in both the data encoding portion of the telemetry, system and also in the modulated RF transmitter.

US Patent No. 6,577,893, discloses the use of CDMA, a form of Spread-Spectrum technology. However, the patent discloses application of a Spread-Spectrum technique directly onto an RF carrier, not onto an audio sub-carrier which in turn modulates an RF carrier as utilized in various embodiments of the present invention. An audio signal, as used herein, is a lower frequency signal which is applied as modulation onto a higher frequency signal. An audio sub-carrier is preferably a fixed-frequency audio signal that is applied as modulation onto a higher frequency signal. In an embodiment, another signal may be applied as modulation to the sub-carrier before the resulting modulated sub-carrier is applied as modulation to an RF carrier. In an embodiment, however, a sub-carrier may have a varying frequency, the variation in frequency being used, for example, to transmit information. Traditionally an audio signal has a frequency range of 20 Hz to 20 KHz. However, circuits designed for audio use may be extended in many cases to operate with signals of much higher frequencies than 20 KHz and/or lower frequencies than 20 Hz.

Medical telemetry data has been transmitted using one or more audio sub-carriers to modulate an RF transmitter. An analog form of this multi-channel data transmission scheme was disclosed by Klein et al. "A Low-Powered 4-Channel Physiological Radio Telemetry System for Use in Surgical Patient Monitoring" IEEE Trans. Biomed. Eng BME-23, #6, Nov. 1976. Embodiments of the present invention, however, encode digital binary data onto the sub-carriers.

Also, Spread-Spectrum techniques have been applied to audio frequency carriers as part of an underwater acoustic communication system. See, for example, Lee Freitag, et al., "Analysis of Channel Effects on Direct-Sequence and Frequency-Hopped Spread-Spectrum Acoustic Communication" IEEE Journal of Oceanic Engineering, Vol., 26, No. 4, October 2001. This publication discloses the wireless transmission of the acoustic signals using sonar techniques. It does not however provide for modulating such acoustic signals onto an RF carrier for RF wireless transmission as provided for in embodiments of the present invention.

Embodiments of the present invention apply Spread-Spectrum techniques to an audio carrier, and then, in turn, utilize the audio Spread-Spectrum signal as modulation onto an RF carrier. Compared to conventional RF Spread-Spectrum data transmission, embodiments of the present invention perform the Spread-Spectrum data encoding at a lower frequency, such as 100 to 1000 Hz, compared to, for example, 1 MHz typical in conventional systems, thus greatly reducing the power consumption of circuitry that performs the data encoding. Also, with audio sub-carrier Spread-Spectrum, in embodiments of the present invention, amplitude modulation of the RF carrier may be used, whereas conventional RF Spread-Spectrum data transmission generally employs frequency shift keying (FSK) or phase shift keying (PSK) of the RF carrier. Such techniques require additional circuitry between the RF oscillator and the antenna, increasing complexity and power consumption of the transmitter.

For a low-power SAW resonator-stabilized RF oscillator embodiment as shown in Figure 1, amplitude modulation provides superior modulation given constraints of circuit complexity and power consumption. Furthermore, Spread-Spectrum decoding in the receiver depends on an accurate and stable received carrier frequency. The audio carrier employed in an embodiment of the present invention as an RF sub-carrier, may be accurately controlled in frequency by the transmitter.

In contrast, a SAW resonator, which determines the RF carrier frequency, may not be able to be controlled in frequency with sufficient accuracy in some situations to easily accommodate conventional Spread-Spectrum encoded reception, resulting in additional complexity to decode the signal in a receiver. However, in an embodiment of the present invention, a SAW resonator may be trimmed or otherwise formed to provide a desired frequency within a desired band.

In an embodiment in which multiple transmitters are utilized, a plurality of SAW resonators may be provided with two or more frequencies distributed throughout the lot. Thus, differentiation by a receiver between SAW resonators with different frequencies may be provided. Further, in an embodiment, a system may be provided with a receiver that is capable of distinguishing the frequencies of one or more SAW resonators.

In an embodiment of the present invention, an audio sub-carrier may utilize different frequencies for different types of data and/or different types of transmission schemes. For example, one sub-carrier frequency may be utilized to transmit identification data, and an additional sub-carrier frequency may be utilized to transmit data indicative of the level of the measured analyte. In an embodiment, different types of data may be transmitted intermittently or continuously, or one type of data may be transmitted intermittently and another type of data may be transmitted continuously. For example, in an embodiment, identification data (i.e., data that identifies transmission from a particular transmitter) may be sent intermittently, whereas analyte-dependent data (i.e., data indicative of the amount of analyte being measured) may be transmitted continuously.

In an embodiment of the present invention in which multiple audio sub-carrier frequencies are utilized, one frequency may be used to identify a different frequency at which analyte-dependent data is being transmitted. In such an embodiment, multiple receivers may be permitted to receive transmissions on the identification frequency, and the identification data may be utilized to direct a desired receiver to listen to a desired frequency on which analyte-dependent data from a desired transmitter is being transmitted.

Figure 2 illustrates an embodiment of the present invention. Following the basic principles of Direct-Sequence-Spread-Spectrum (DSSS), a carrier frequency may be generated, in this case in the audio range, at 202. In an embodiment, a means may be provided to pass this carrier frequency either unaltered, or the polarity of the sub-carrier may be inverted in response to a control signal 204. This may be performed with an exclusive-OR function as shown at 206. The output of exclusive-OR function 206 may be applied as amplitude modulation to the RF oscillator and transmitter 208. Control signal 204 may be generated by combining the bit-serial binary data to be transmitted at 210 with a pseudorandom binary sequence 212 generated at 216. This may be performed using the exclusive-OR function at 214. The pseudorandom binary sequence, as is well known in DSSS theory, may be chosen to satisfy several requirements. In an embodiment of the present invention, the autocorrelation function for the pseudorandom sequence equals a low value, except for time equal to zero. Several classes of useful sequences are known, for example M-codes, Gold Codes or Kasami Codes, each of which are contemplated within the scope of embodiments of the present invention.

Following the basic principles of DSSS, the bit rate for the pseudorandom sequence determines the audio bandwidth of the transmitted signal, centered at the frequency of the audio sub-carrier. In an embodiment of the present invention, the pseudorandom sequence bit rate is less than the sub-carrier frequency, preferably a fraction of the sub-carrier frequency, such as 1/4. Also, in an embodiment of the present invention, the data bit rate is a fraction of the pseudorandom sequence bit rate, such as 1/127. For example, the sub-carrier frequency may be about 4 KHz for a typical low-power transmitter design. In an embodiment of the present invention, the pseudorandom sequence bit rate may be, for example, 1 KHz and the data bit rate may be, for example, 8Hz. In an embodiment, the number of pseudorandom sequence bits per data bit is chosen to equal the number of bits after which the pseudorandom sequence repeats.

As may be appreciated by those skilled in the art, the entire data processing sequence, from digitizing analog sensor data through creation of the modulated sub-carrier ready for amplitude modulation of the RF carrier, may be efficiently performed by a suitably programmed and configured microprocessor such as a Microchip PIC12F675, for example. Alternatively, discrete logic may be designed, as well as numerous other implementations according to embodiments of the present invention.

In an embodiment, a receiver for reception of DSSS signals applied to an audio sub-carrier, as described herein, may be configured for the reception of amplitude modulated signals. The receiver may have an RF bandwidth sufficient to receive signals from multiple transmitters which are, for example, nominally on the same frequency, but which may actually be on slightly different frequencies because of manufacturing tolerances in the SAW resonators, or specially designed variations in the SAW resonators. In an embodiment of the present invention, a SAW resonator for 915 MHz may have tolerances of +-75KHz and thus a receiver RF bandwidth of, for example, 150 KHz may be utilized. According to an embodiment of the present invention, the receiver audio bandwidth may be sufficient to pass the sub-carrier frequency and sidebands generated by the DSSS process. For example, with a sub-carrier at 4 KHz and a pseudorandom sequence rate of 1 KHz, a band-pass from 3 KHz to 5 KHz may be sufficient.

In an embodiment of the present invention, the received audio signal spectrum may be centered at the transmitted sub-carrier frequency. The encoded data may be recovered by providing a means to pass the received audio signal either unaltered, or inverted in response to a control signal. In an embodiment, the control signal may be a pseudorandom binary bit sequence identical to that used in the transmitter. When the timing of the receiver pseudorandom sequence matches that of the desired transmitter, the transmitted binary data may be recovered.

Further, in an embodiment of the present invention, a receiver may be provided with a scanning function to scan the desired frequency range. In an embodiment of the present invention, a receiver may lock-on to a received frequency, whether that frequency is predefined or that frequency is transmitted and identified during an initialization process.

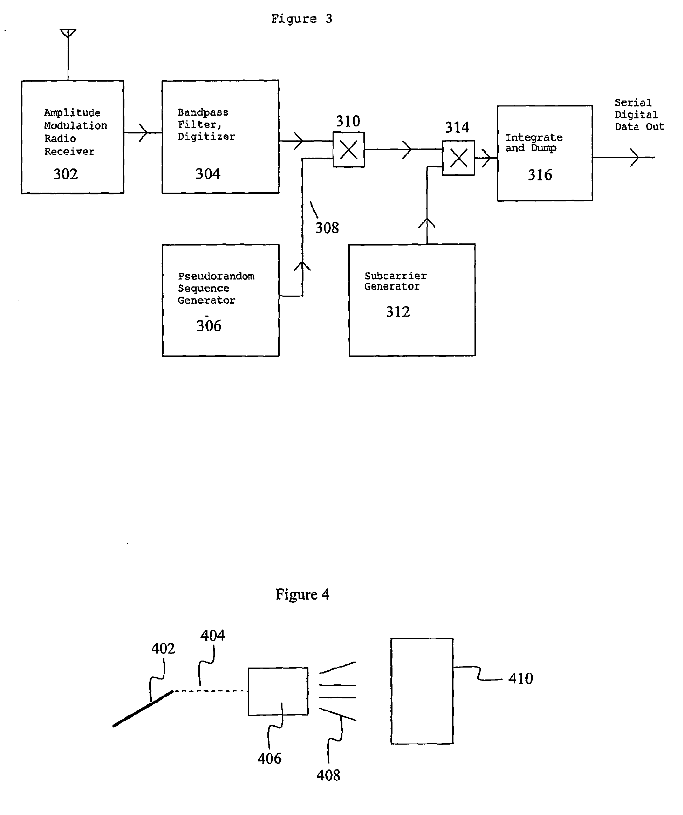

In the embodiment shown in Figure 3, an amplitude modulation receiver tuned to the RF carrier frequency is shown in 302. In an embodiment, the analog audio output of the receiver may be band-pass filtered to remove noise outside the expected frequency range for the desired signals. The analog signal may then be digitized at 304, to allow digital processing, for example, in a suitably programmed and configured microprocessor.

A pseudorandom sequence 308, matching that used at the transmitter, may be generated at 306. The digitized and filtered received signal from 304 may be combined with the pseudorandom signal 308 in multiplier 310. A digital audio sub-carrier, matching in frequency that used in the transmitter, may be generated at 312. This sub-carrier may be combined with the output of multiplier 310 in multiplier 314. The resulting signal may be integrated over one data bit interval and the result may be compared against a threshold to detect a binary 1 or 0 in the received data. This may be performed by the integrate-and-dump circuit at 316. The resulting serial data stream may then be ready for conventional de-serialization and further data processing.

In an embodiment, when multiple transmitters are present, the transmitters may operate independently and the timing of the pseudorandom sequence generated by each transmitter may be uncorrelated. The low correlation between pseudorandom sequences of the multiple transmitters may occur when all transmitters use the same pseudorandom sequence but with different timing, or when each transmitter uses a unique pseudorandom code. Given the autocorrelation property of the pseudorandom code, a receiver may align the timing of its pseudorandom code to match that of the desired transmitter. Transmission from other transmitters may be rejected as their contributions to decoder output average out to zero. Similarly, interfering signals, including "beat notes" between the carrier frequencies of several transmitters on slightly different frequencies, may be rejected since they tend to have a cross-correlation to the pseudorandom sequence close to zero.

In an embodiment of the present invention, a receiver may perform a search process during which it may align its pseudorandom sequence in turn to an available transmitter. The receiver may then determine the identity of each transmitter and then choose the pseudorandom sequence timing that aligns with the desired transmitter for reception.

In an embodiment, such an algorithm may adjust (increment or decrement) the timing of the receiver pseudorandom sequence in increments of one bit until a correlation is detected with the received signal. In an embodiment, the phase of the receiver pseudorandom sequence may be adjusted within the bit interval to maximize the correlation. In an embodiment, data may then be received and examined by the receiver to determine if it has synchronized to the desired transmitter.

In an embodiment of the present invention, when several transmitters are operating within range of the receiver, their respective transmissions may be received with widely different signal levels, based on distance between transmitter and receiver, for example. Since the auto correlation properties of the DSSS pseudorandom sequences are not perfect, a strong signal may create some interference with the reception of a weak desired signal. In prior attempts, this problem had been addressed by providing a means for the receiver to control the power of the transmitters. However, this requires that a receiver be provided at each transmitter, increasing the complexity and power consumption of the transmitting system. Also, in the case of ambulatory medical telemetry, there may in fact be several receivers as well as several transmitters. Each receiver may have a different requirement for signal strengths from the various transmitters which may conflict with the requirements of other receivers. In an embodiment, individual transmitters may be configured to transmit data using differing RF power levels. The RF power level chosen for each transmission may be determined randomly and independently for each individual transmitter, without knowledge of other transmitters within receiving range. Therefore, in case of interference with a transmission from a weak signal by the presence of a stronger signal, in a subsequent transmission, the strong interfering transmission may be weaker and/or the weaker desired signal may be stronger. Because of the random nature of the relative power levels between the desired and interfering signals, the signal strength of the desired signal may be favorable relative to the interfering signal from time to time, on a random basis, facilitating transmission of data. Furthermore, the average power level for each transmitter may be lower than its maximum power level, contributing to reduced average power consumption and longer battery life.

In embodiments, antenna redundancy may be used to increase data transmission (whether continuous or intermittent) success between transmitter(s) and receiver(s).

In an embodiment of the present invention, an RF transmitter may utilize one or more antennas to facilitate transmission of the RF signal. Depending on the relative orientations and locations of the transmitter and receiver, the transmitter may transmit in a preferred direction to increase signal strength at the receiver. Objects in the vicinity of the transmitter and receiver may reflect a portion of the RF signal and cause destructive interference and a loss in received signal strength. In an embodiment, multiple transmit antennas optimized for transmissions with differing orientations and/or polarizations may thus provide increased RF signal strength at the receiver. Increased RF signal strength at the receiver may increase the quality of data recovered and reduce data transmission errors.

In an embodiment of the present invention, an RF receiver may utilize one or more antennas to facilitate reception of the RF signal. An RF signal received from the transmitter may be received from one of multiple directions, depending on the relative orientations and locations of the transmitter and receiver. An RF signal may be received from the transmitter from several directions at once when, for example, the transmitted signal is reflected from objects in the vicinity of the transmitter or receiver. Destructive interference between multiple instances of the RF signal may cause a drop in received signal strength and a loss of received data. In an embodiment, the receiver may receive inputs from multiple receive antennas with differing orientations and/or polarizations. In an embodiment, the receiver may select the RF signal from one or more of the multiple antennas which provide the strongest signal and the most reliable source of data.

In an embodiment, data may be encoded using an error-correcting code, such as, for example, a Reed-Soloman code, before creating the Spread-Spectrum audio signal. In an embodiment, convolution encoding such as Viterbi Coding or Turbo Coding may be employed to provide redundancy in the transmitted data. The receiver may then be able to recover error-free data despite a limited number of reception errors. Such errors may occur, for example, if the transmitted signal is weak at the receiver and/or has been corrupted with random noise.

Common error correcting digital information coding techniques add redundancy to the transmitted data message to facilitate recovery of the original transmitted data despite the loss of some number of data bits during the transmission process. For example, a data message may contain N bits. An additional M bits are added to the message before transmission. The content of the bits may be determined by one of several algorithms understood by those skilled in the art. In an embodiment, the receiving device receives the message of N+M bits and employs an algorithm to extract the message of N bits. The algorithms allow the receiver to determine if one or more of the N+M bits were received in error. Also, if the number of erroneous bits is not excessive, in an embodiment, an algorithm may provide a means to recover the original message despite the errors.

In convolution codes, for example, M may equal N and a message of N bits may be transmitted as a message of M+N or 2N total bits. Each of the transmitted 2N bits may be defined by an algorithm in which its value depends on the values of several of the original N message bits. Consequently, each of the original N message bits influences the value of several of the 2N transmitted bits. For example, each of the original message bits may influence the value of 3, 5 or 7 of the transmitted bits. If one or more of the transmitted bits is received in error, the convolution decoding algorithm in the receiver may still be able to reconstruct the original N message bits because each of these N bits may be represented in more than one of the transmitted bits. Trellis decoding is a common algorithm for decoding a convolution coded message at a receiver and may be utilized in an embodiment of the present invention.

Reed-Soloman coding also employs an additional M data bits transmitted along with the N message bits. Using the mathematical properties of Galois Fields, the N bit message may be represented as an N-dimensional vector space. The algorithm provides a method to map this N-dimensional vector to an N+M-dimensional vector space and to map N+M-dimensional vectors back to the N-dimensional original vector space that represents the original message. The algorithms have unique properties that provide that for a given N-bit message, all N+M bit received messages, with all possible errors up to a specified limit, map back to the original transmitted N-bit message vector. The allowable number of errors that may be corrected in a given message is a function of M, the number of additional error correcting bits that the algorithm may append to the original N message data bits.

Other error correcting message coding techniques are well understood by those skilled in the art, see, for example, W. Wesley Peterson, and E. J. Weldon, Jr., Error Correcting Codes, Second Edition, Cambridge Mass: 1972, The IVIIT Press; and M. C. Valenti and J. Sun, The UMTS Turbo Code and an Efficient Decoder Implementation Suitable for Software-Defined Radios, International Journal of Wireless Information Networks, Vol. 8, No. 4, October 2001.

In an embodiment, data may be transmitted multiple times (whether continuously or intermittently), allowing the receiver to correctly decode data despite occasional transmission errors. Transmission repeats or duplication may be performed for any desired number of data points or data words, for example every data point may be transmitted 2 or 3 times consecutively, or every series of 10 data points/words may be repeated, etc. In an embodiment, data may be transmitted in a continuous stream allowing a receiver to disregard an erroneous transmission of a data point or data word without jeopardizing the real-time indication of a particular analyte condition.

In an embodiment, a transmitter may attach error detection bits to each transmission. Error detection bits may be generated by the transmitter using any of several well-known algorithms. Subsequently, the receiver may apply the corresponding algorithm to the received data and error detection bits to determine if all data bits were received correctly. Common error detection algorithms are less complex than error correction algorithms, but nevertheless allow the receiver to identify corrupted data which may be discarded. For example, the CRC8 algorithm is described in ITU-T I.432.1 (International Telecommunication Union), B-ISDN user-network interface-Physical layer specification: General characteristics, 02/1999, which may be used in an embodiment of the present invention.

In an embodiment, transmitted data may be compressed to reduce the number of bits that must be received without errors and still be a satisfactory data transmission. As an example, in an embodiment, only changes in a data value may be transmitted. In an embodiment, run-length coding of data may be incorporated.

In an embodiment of the present invention, a transmitter may be configured to continuously transmit data at two or more different power levels. A transmitter may operate at a higher power level for only a portion of the time, avoiding possible interference with other transmitters and keeping transmitter power consumption low. However, the occasional high power transmission boost assures that the data may be received with some regularity even when the receiver is in an unfavorable location for reception from the transmitter. Under favorable reception conditions, the receiver may receive the data continuously. In an embodiment, the timing of the various transmit power modes may be independent and/or random for individual transmitters, greatly reducing the probability that two potentially interfering transmitters may both be transmitting in a high power mode simultaneously.

In an embodiment of the RF transmitter such as shown in Figure 1, the transmitted RF power may readily be adjusted by providing a means to vary the emitter resistor on the transistor, or otherwise vary the transistor collector current.

In an embodiment, another form of Spread-Spectrum data transmission may be employed. For example, Frequency-Hopping-Spread-Spectrum may be applied to an audio sub-carrier or directly applied to the RF carrier. Following the well-known principles of frequency hopping spread spectrum, the RF transmit frequency may be periodically changed or varied. The receive frequency must be similarly changed to correspond with the transmit frequency. In an embodiment, frequency hopping spread spectrum provides reliable transmission although noise or interference may prevent RF communication on some transmit frequencies. Also, frequency hopping spread spectrum may provide improvements in transmission reliability when a frequency-dependent increase in signal loss is present An example of such a frequency-dependent signal loss is multi-path fading. An embodiment of frequency hopping spread spectrum provides a means for the RF receiver to adjust its receive frequency as the transmitter changes the transmit frequency. The sequence of transmit frequencies may be predetermined and known by both the receiver and transmitter, or the transmitter may transmit to the receiver the next frequency that will be used. In an embodiment, the transmitter may utilize adaptive frequency hopping spread spectrum. In this case, the transmitter may attempt reception on a proposed transmit frequency to assess the presence of interference before using the frequency for transmission. An embodiment of frequency hopping spread spectrum also provides a means for the transmitter and receiver to start operation on the same frequency and at the start of a repeated and predetermined sequence of frequencies, see, for example, Haykin, Simon, Communication Systems, Wiley, 2001.

An embodiment of the present invention may apply frequency hopping principles to vary the transmitted frequency of the audio sub-carrier, with or without varying the frequency of the transmitted RF carrier. Application of frequency hopping principles to an audio sub-carrier rather than the transmitter RF carrier may prove beneficial in an embodiment of a low-power continuous RF data transmitter.

In an embodiment, an alarm indicating one or more medically significant conditions may be provided as part of the sensor control unit attached to a patient. In this embodiment, the patient may be notified of alarm conditions without relying on data transmission to a remote data display and recording unit. Thus, in an embodiment, data may be continuously acquired and transmitted, and whether or not within a suitable reception range between a transmitter and a receiver, a sensor control unit, or other similar device, may be configured to provide an indication of a current analyte condition. Such an indication may comprise, for example, an audible alarm or vibration indicating a dangerous condition.

Figure 4 illustrates an exemplary sensing system utilizing telemetry in accordance with an embodiment of the present invention. Sensor 402 may be inserted or implanted into a body to measure analyte (glucose, lactate, etc.) within a body, such as within the subcutaneous tissue or within the blood. Sensor 402 may be connected (404) in a variety of ways to an on-skin or external unit 406. For example, a sensor 402 may be connected to unit 406 by a direct hard-wired electrical connection, a two-part mateable electrical connection, or telemetrically, such as using RF transmission, inductive coupling, infrared, etc. Unit 406 may in turn communicate data telemetrically (408) to a monitoring unit 410. Monitoring unit 410 may be a table top unit, a handheld unit, a wearable unit, a PDA, a wrist watch, a cell phone, etc. Data may be transmitted from unit 406 to monitoring unit 410 as described in various embodiments above.

Although certain embodiments have been illustrated and described herein for purposes of description of the preferred embodiment, it will be appreciated by those of ordinary skill in the art that a wide variety of alternate and/or equivalent embodiments or implementations calculated to achieve the same purposes may be substituted for the embodiments shown and described without departing from the scope of the present invention. Those with skill in the art will readily appreciate that embodiments in accordance with the present invention may be implemented in a very wide variety of ways. This application is intended to cover any adaptations or variations of the embodiments discussed herein. Therefore, it is manifestly intended that embodiments in accordance with the present invention be limited only by the claims and the equivalents thereof.

## Claims

1. A computer implemented method of providing a transmission signal indicative of a level of an analyte in a body of an animal, comprising:
acquiring a variable voltage indicative of the level of the analyte in the body of the animal;
converting said variable voltage to digital data;
digitally modulating a first audio sub-carrier signal using said digital data;
modulating an RF carrier signal using the digitally modulated audio sub-carrier signal to create for transmission by a radio frequency (RF) transmitter of a sensor control unit, a transmission signal indicative of the level of the analyte in the body of the animal.

2. The method of claim 1, wherein said modulating an RF carrier signal comprises amplitude modulating or frequency modulating said RF carrier signal.

3. The method of claim 1, wherein said transmission signal further comprises:
a. data identifying the transmitting transmitter, or
b. at least one of error detection bits and error correction bits, or
c. data which is transmitted continuously, or
d. a convolution code being applied to said transmission for error correction.

4. The method of claim 1, wherein said digitally modulating a first audio sub-carrier signal further comprises applying a spread spectrum technique to said first audio sub-carrier signal, and advantageously, wherein said spread spectrum technique comprises direct sequence spread spectrum or frequency hopping spread spectrum.

5. The method of claim 1, wherein said RF carrier signal is generated by a surface acoustic wave resonator configured with a transistor oscillator.

6. The method of claim 1, wherein a second audio sub-carrier signal is provided having:
a. a frequency different from a frequency of said first audio sub-carrier, wherein said second audio-sub carrier frequency is utilized to transmit identification data of the transmitting transmitter, or
b. a frequency different from a frequency of said first audio sub-carrier signal, wherein said second audio-sub carrier frequency is utilized to indicate the frequency at which said first audio sub-carrier is transmitted, and advantageously, wherein said identification data is transmitted intermittently.

7. The method of claim 1, wherein said modulating an RF carrier signal comprises:
a. frequency modulating said RF carrier signal by electronically switching a loading capacitor across a surface acoustic wave resonator, or
b. amplitude modulating said RF carrier signal by adjusting a base bias voltage being provided to a transistor oscillator.

8. An apparatus comprising:
an analyte sensor (402) adapted for full or partial implantation into a body of an animal for acquisition of data indicative of a level of an analyte in the body; and
a sensor control unit coupled to said sensor, said sensor control unit comprising a radio frequency (RF) transmitter, said RF transmitter adapted to transmit an RF carrier signal and to receive as amplitude modulation of the RF carrier signal an audio sub-carrier signal, wherein the audio sub-carrier signal has been digitally modulated and comprises digital data derived from the acquired data indicative of a level of an analyte in the body.

9. The apparatus of claim 8, further comprising:
a. a signal converter to convert a variable voltage acquired from said sensor and indicative of a level of analyte in the body to the digital data, or
b. a surface acoustic wave resonator (108) configured with a transistor oscillator, wherein said surface acoustic wave resonator is configured to generate the RF carrier signal, or
c. a battery (110) to power said transmitter, wherein said battery is adapted to be recharged, or
d. a plurality of transmit antennas (106) coupled to said sensor control unit, wherein said plurality of transmit antennas are each independently adapted for positioning in a variety of orientations to increase transmission strength and/or accuracy.

## Patentansprüche

1. Computerimplementiertes Verfahren zum Bereitstellen eines Sendesignals, das einen Pegel eines Analyten im Körper eines Tieres indiziert, umfassend:
Ein Erfassen einer variablen Spannung, die den Pegel des Analyten im Körper des Tieres indiziert;
Konvertieren der variablen Spannung in digitale Daten;
Digitales Modulieren eines ersten Ton-Zwischenträgersignal-Signals unter Verwendung der digitalen Daten;
Modulieren eines RF-Trägersignals unter Verwendung des digital modulierten Ton-Zwischenträgersignalsignals zur Schaffung eines Übertragungssignals für eine Übertragung mittels eines Radiofrequenz-(RF)-Transmitters einer Sensor-Steuereinheit, welches Signal den Pegel des Analyten des Körper des Tieres indiziert.

2. Verfahren nach Anspruch 1, wobei das Modulieren eines RF-Trägersignals eine Amplitudenmodulation oder Frequenz-Modulation des RF-Trägersignals umfasst.

3. Verfahren nach Anspruch 1, wobei das Übertragungssignal ferner umfasst:
a. Daten, die den Übertragungstransmitter identifizieren, oder
b. zumindest ein Fehlerermittlungs-Bit oder Fehlerkorrektur-Bit, oder
c. Daten, die kontinuierlich übertragen werden, oder
d. einen Konvolutions-Code, der auf die Übertragung zur Fehlerkorrektur angewendet wird.

4. Verfahren nach Anspruch 1, wobei das digitale Modulieren eines ersten Ton-Zwischenträgersignalsignals ferner das Anwenden einer Spektralverteilungs-Technik auf das erste Ton-Zwischenträgersignalsignal umfasst, und vorteilhafterweise weist die Spektralverteilungstechnik eine PN-Spektrumspreizmodulation oder eine Frequenz-Sprung-Spektrumspreizmodulation auf.

5. Verfahren nach Anspruch 1, wobei das RF-Trägersignal durch einen akustischen Oberflächenwellen-Resonator generiert wird, der mit einem Transistor-Oszillator figuriert ist.

6. Verfahren nach Anspruch 1, wobei ein zweites Ton-Zwischenträgersignal vorgesehen ist, mit:
a. einer von einer Frequenz des ersten Ton-Zwischenträgers unterschiedlichen Frequenz, wobei die Frequenz des zweiten Ton-Zwischenträgers verwendet wird, um Identifikationsdaten des Übertragungstransmitters zu übertragen, oder
b. einer von einer Frequenz des ersten Ton-Zwischenträgersignals unterschiedlichen Frequenz, wobei die Frequenz des zweiten Ton-Zwischenträgers verwendet wird, um die Frequenz aufzuzeigen, zu der der erste Ton-Zwischenträger übertragen wird, und vorteilhafterweise werden die Identifikationsdaten intermittierend übertragen.

7. Verfahren nach Anspruch 1, wobei das Modulieren eines RF-Trägersignals aufweist:
a. eine Frequenzmodulation des RF-Trägersignals durch ein elektronisches Schalten einer Ladekapazität über einen akustischen Oberflächenwellen-Resonator, oder
b. eine Amplitudenmodulation des RF-Trägersignals durch Einstellen einer Basisvorspannung, die an einem Transistor-Oszillator anliegt.

8. Vorrichtung, aufweisend:
einen Analyt-Sensor (402) zur vollständigen und teilweisen Implantation in einen Körper eines Tieres zur Erfassung von Daten, die einen Pegel eines Analyten in dem Körper indizieren, und
eine Sensor-Steuereinheit, die an dem Sensor angeschlossen ist, wobei die Sensor-Steuereinheit einen Radiofrequenz-(RF)-Transmitter aufweist, welcher RF-Transmitter zur Übertragung eines RF-Trägersignals und zum Empfang eines Ton-Zwischenträger-Signals als eine Amplitudenmodulation des RF-Trägersignals bestimmt ist, wobei das Ton-Zwischenträgersignal digital moduliert wurde und digitale Daten umfasst, die aus den erfassten Daten abgeleitet werden, die auf einen Pegel eines Analyten im dem Körper schließen lassen.

9. Vorrichtung nach Anspruch 8, ferner aufweisend:
a. ein Signalwandler zum Konvertieren einer variablen Spannung, die von dem Sensor erfasst wird und einen Pegel eines Analyten in dem Körper anzeigt, in digitale Daten, oder
b. einen akustischen Oberflächenwellen-Resonator (108), der mit einem Transistor-Oszillator konfiguriert ist, wobei der akustische Oberflächenwellen-Resonator zur Erzeugung des RF-Trägersignals konfiguriert ist, oder
c. eine Batterie (110) zur Stromversorgung des Transmitters, wobei die Batterie zur Wiederaufladung bestimmt ist, oder
d. eine Mehrzahl an Übertragungsantennen (106), die an der Sensor-Steuereinheit angeschlossen sind, wobei die Mehrzahl der Übertragungsantennen zueinander unabhängig zur Positionierung in einer Vielzahl von Orientierungen bestimmt ist, um die Übertragungsstärke und/oder Genauigkeit zu erhöhen.

## Revendications

1. Procédé informatique consistant à fournir un signal de transmission indicatif du niveau d'un analyte dans le corps d'un animal, qui comprend :
l'acquisition d'une tension variable indicative du niveau de l'analyte dans le corps de l'animal ;
la conversion de ladite tension variable en données numériques ;
la modulation numérique d'un premier signal de sous-porteuse audio en utilisant lesdites données numériques ;
la modulation d'un signal de porteuse RF en utilisant le signal de sous-porteuse audio modulé numériquement afin de créer, en vue de sa transmission par un émetteur radiofréquence (RF) d'une unité de commande de détecteur, un signal de transmission indicatif du niveau de l'analyte dans le corps de l'animal.

2. Procédé selon la revendication 1, dans lequel ladite modulation d'un signal de porteuse RF comprend la modulation en amplitude ou la modulation en fréquence dudit signal de porteuse RF.

3. Procédé selon la revendication 1, dans lequel ledit signal de transmission comprend en outre :
a. des données identifiant l'émetteur de transmission, ou
b. au moins l'un de bits de détection d'erreurs et de bits de correction d'erreurs, ou
c. des données qui sont transmises en continu, ou
d. un code de convolution appliqué à ladite transmission pour corriger les erreurs.

4. Procédé selon la revendication 1, dans lequel ladite modulation numérique d'un premier signal de sous-porteuse audio comprend en outre l'application d'une technique d'étalement de spectre audit premier signal de sous-porteuse audio et, avantageusement, dans lequel ladite technique d'étalement de spectre comprend un étalement de spectre par séquence directe ou un étalement de spectre par saut de fréquence.

5. Procédé selon la revendication 1, dans lequel ledit signal de porteuse RF est généré par un résonateur à onde acoustique de surface configuré avec un oscillateur à transistor.

6. Procédé selon la revendication 1, dans lequel un second signal de sous-porteuse audio est fourni avec :
a. une fréquence différente de celle de ladite première sous-porteuse audio, ladite seconde fréquence de sous-porteuse audio étant utilisée pour transmettre les données d'identification de l'émetteur de transmission, ou
b. une fréquence différente de celle dudit premier signal de sous-porteuse audio, ladite seconde fréquence de sous-porteuse audio étant utilisée pour indiquer la fréquence à laquelle ladite première sous-porteuse audio est transmise, et, avantageusement, lesdites données d'identification étant transmises de manière intermittente.

7. Procédé selon la revendication 1, dans lequel ladite modulation d'un signal de porteuse RF comprend :
a. une modulation en fréquence dudit signal de porteuse RF en commutant électroniquement un condensateur de charge au sein d'un résonateur à onde acoustique de surface, ou
b. une modulation en amplitude dudit signal de porteuse RF en ajustant une tension de polarisation de base fournie à un oscillateur à transistor.

8. Appareil comprenant :
un détecteur d'analyte (402) adapté pour être implanté entièrement ou partiellement dans le corps d'un animal afin d'acquérir des données indicatives du niveau d'un analyte dans le corps ; et
une unité de commande de détecteur couplée audit détecteur, ladite unité de commande de détecteur comprenant un émetteur radiofréquence (RF), ledit émetteur RF étant adapté pour transmettre un signal de porteuse RF et pour recevoir, en guise de modulation en amplitude du signal de porteuse RF, un signal de sous-porteuse audio, le signal de sous-porteuse audio ayant été modulé numériquement et comprenant des données numériques dérivées des données acquises indicatives du niveau d'un analyte dans le corps.

9. Appareil selon la revendication 8, comprenant en outre :
a. un convertisseur de signal destiné à convertir une tension variable acquise à l'aide dudit détecteur et indicative du niveau d'analyte dans le corps en données numériques, ou
b. un résonateur à onde acoustique de surface (108) configuré avec un oscillateur à transistor, ledit résonateur à onde acoustique de surface étant configuré pour générer le signal de porteuse RF, ou
c. une batterie (110) destinée à alimenter ledit émetteur, ladite batterie étant adaptée pour être rechargée, ou
d. une pluralité d'antennes de transmission (106) couplées à ladite unité de commande de détecteur, ladite pluralité d'antennes de transmission étant chacune adaptées indépendamment pour être placées selon plusieurs orientations afin d'augmenter l'intensité et/ou la précision de la transmission.
